# NEUE EUROPÄISCHE PATENTSCHRIFT

(11) **EP 1 038 552 B2**
(45) Veröffentlichungstag und Bekanntmachung des Hinweises auf die Entscheidung über den Einspruch: **20.11.2013**
(45) Hinweis auf die Patenterteilung: 16.02.2005
(21) Anmeldenummer: 00103885.0
(22) Anmeldetag: 24.02.2000
(51) Int. Cl.: A61P 7/08, A61K 33/00, A61M 1/16, A61M 1/28, A61K 9/08, A61K 47/26, A61K 47/12

(54) **Lösung, insbesondere für Hämo- oder Peritonealdialyse, sowie Verfahren zu deren Herstellung**
Solution, in particular a hemodialysis or peritoneal dialysis solution, and method for producing same
Solution, en particulier une solution de hemodialyse ou de dialyse peritoneale, ainsi que son procédé de fabrication

(30) Priorität: 22.03.1999 DE 19912850
(43) Veröffentlichungstag der Anmeldung: 27.09.2000
(73) Patentinhaber: Fresenius Medical Care Deutschland GmbH, 61352 Bad Homburg v.d.H. (DE)
(72) Erfinder: Knerr, Thomas, 66606 St. Wendel (DE)
(74) Vertreter: Laufhütte, Dieter

(56) Entgegenhaltungen:
- EP-A- 0 564 672
- EP-A1- 1 008 341
- WO-A-00/57833
- WO-A-97/05852
- WO-A1-93/09820
- WO-A1-96/01118
- WO-A1-97/07837
- WO-A1-99/27885
- WO-A1-93//19792
- WO-A2-91/08008
- US-A- 4 489 535
- US-A- 5 616 248
- US-A- 5 827 820
- DATABASE CAPLUS [Online] CHEMICAL ABSTRACTS SERVICE, COLUMBUS, OHIO, US; TREZECIAK, MARZENNA ET AL: "Effect of the electrolyte comonents of peritoneal dialysis solutions on stability of glucose" Database accession no. 1990:25482 XP002225451
- TREZECIAK , MARZENNA ET AL.: 'Acta poloniae pharmaceutica', Bd. 46(2), 1989, THE BRITISH LIBRARY Seiten 174 - 178
- 'The international journal of artificial organs', Bd. 8, 1985, WICHTIG EDITORE vol. 'Bicarbonate solutions for peritoneal dialysis: a reality', Seiten 57 - 58
- 'British Pharmacopoeia', Bd. 2, 1998 Seiten 1862 - 1863
- Examination Report of 18.09.2001 in relation to prosecution of WO-A-99/27885
- Examination Report of 29.11.2002 in relation to prosecution of WO-A-99/27885

## Beschreibung

Die Erfindung betrifft eine Lösung, insbesondere für die Hämo- oder Peritonealdialyse, sowie ein Verfahren zu deren Herstellung.

Die wichtigsten funktionellen Bestandteile von Hämodialyse- oder Peritonealdialyselösungen sind Elektrolyte, die vorzugsweise Calcium-, Natrium-, Magnesiumsowie Chloridionen umfassen, ein Puffersystem sowie ein geeignetes Osmotikum.

Als Puffersystem wird üblicherweise ein Bicarbonatpuffer verwendet, der den Vorteil einer guten physiologischen Verträglichkeit aufweist, der jedoch je nach pH-Wert der Lösung im alkalischen Milieu teilweise in Carbonatform vorliegt und im sauren Bereich mit CO₂ im Gleichgewicht steht. Neben Bicarbonat sind auch andere Puffer einsetzbar, die in dem physiologischen pH-Bereich von ca. 7 eine ausreichende Pufferwirkung haben. In Frage kommen Lactat oder Pyruvat, die im Körper leicht zu Bicarbonat abgebaut werden können.

Als Osmotikum wird üblicherweise Glucose verwendet, die im Bereich der angestrebten Osmolarität gut verträglich ist.

Bei der Herstellung einer die funktionellen Bestandteile enthaltenden Dialyselösung besteht ein wesentliches Problem darin, daß Bicarbonat und Calcium in der Regel getrennt gelagert werden müssen, um die Entstehung eines unlöslichen Calcium carbonat-Niederschlags zu verhindern. Zwar kann im sauren Bereich ein derartiger Niederschlag vermieden werden, jedoch stellt sich das Problem, daß das Bicarbonat - CO₂ - Gleichgewicht im sauren Bereich auf der Seite des CO₂ liegt. Der daraus resultierende relativ hohe CO₂-Partialdruck erfordert entsprechend eine Beutelfolie mit großer, d.h. wirksamer CO₂-Barriere.

Wird als Osmotikum Glucose eingesetzt, kann es bei der Hitzesterilisation der entsprechenden Lösung ferner dazu kommen, daß Glucose mit anderen Bestandteilen der Lösung reagiert und entsprechend Abbauprodukte gebildet werden, die auf den Organismus schädigend einwirken können. Hierbei ist insbesondere die Reaktion von Glucose mit Lactat zu erwähnen, das ebenfalls als Puffer Verwendung findet.

Es ist daher beispielsweise aus der WO 93/09820 bekannt, Glucose und die weiteren Bestandteile der Dialyselösung getrennt der Hitzesterilisation zuzuführen. Hier ist ein Doppelkammergefäß offenbart, bei dem in einem größeren Kompartiment außer Glucose alle wesentlichen Bestandteile der Dialyselösung aufgenommen sind, während die Glucose oder glucoseähnliche Komponenten in einem zweiten Kompartiment aufbewahrt werden. Dadurch kann das Entstehen von Abbauprodukten von Glucose während der Hitzesterilisation wirksam verhindert werden. Nach der Hitzesterilisation werden beide Kompartimente gemischt und die Lösung in bekannter Weise zur Hämodialyse bzw. Peritonealdialyse verwendet.

In der EP 0 613 688 A1 wird eine Dialysierflüssigkeit offenbart, die aus mehreren einzelnen Konzentraten besteht. Hierbei ist ein einheitliches und standardisiertes Grundkonzentrat sowie ein individuell ausgewähltes Zusatzkonzentrat vorgesehen. Das Grundkonzentrat enthält Natriumchlorid und Natriumbicarbonat. Das Zusatzkonzentrat enthält vorzugsweise die weiteren Elektrolyte sowie Glucose. Neben der Trennung der Glucose vom Puffersystem und der damit einhergehender Verringerung der Entstehung unerwünschter Abbauprodukte ergibt sich der Vorteil, daß die Zusammensetzung der Dialyselösung an die jeweils vorliegenden Bedürfnisse des Patienten durch das Individuell ausgewählte Zusatzkonzentrat angepaßt werden kann. Hierdurch wird es möglich, beispielsweise Kalium-, Calcium- und Magnesiumsalze sowie Glucose in individuell angepaßten Dosierungen zu verabreichen.

Die EP 0 402 505 A1 offenbart ein Verfahren und eine Vorrichtung zur kontinuierlichen zyklischen Peritonealdialyse, bei der eine Glucosepumpe an die zum Patienten führende Zulaufleitung für die Dialyselösung angeschlossen ist. Je nach Förderhöhe der Glucosepumpe kann ein unterschiedlicher Gehalt an Glucose in der dem Patienten zugeführten Peritonealdialyselösung eingestellt werden. Die Einstellung des zu verabreichenden Glucosegehaltes wird aufgrund der dem Patienten entzogenen Ultrafiltratmenge vorgenommen.

Den vorbekannten Verfahren zur Herstellung von Dialyselösungen ist es nachteilig, daß durch die Mischung zweier Einzellösungen zwar unterschiedliche Konzentrationen oder auch Profile von Komponenten, insbesondere von Glucose in der fertigen Mischung realisiert werden können, jedoch führt die Mischung der Einzellösungen stets auch zu einer entsprechenden Änderung der Konzentration der weiteren Bestandteile. Wird beispielsweise bei dem aus der EP 0 402 505 A1 bekannten Verfahren Glucose zugemischt, ergeben sich entsprechend des Mischungsverhältnisses auch Konzentrationsänderungen der anderen Bestandteile der Dialyselösung, beispielsweise der Elektrolyte bzw. des pH-Wertes.

Es ist daher die Aufgabe der vorliegenden Erfindung eine Lösung, insbesondere für die Hämo- oder Peritonealdialyse, sowie ein Verfahren zu deren Herstellung an die Hand zu geben, mittels dessen die Glucosekonzentration in beliebiger Weise ohne Beeinflussung der Konzentrationen anderer Bestandteile variiert werden kann.

Diese Aufgabe wird durch eine Lösung mit den Merkmalen des Anspruchs 1 gelöst.

Durch eine derartige Lösung wird der Vorteil erreicht, daß die Glucosekonzentration entsprechend dem Mischungsverhältnis aus erster und zweiter Einzellösung beliebig verändert werden kann, ohne daß sich dabei die Konzentrationen der weiteren Bestandteile ändern.

Ferner weist die erfindungsgemäße Lösung den Vorteil auf, daß die Calciumionen sowie das Bicarbonat in getrennten Kompartimenten aufgenommen sind, so daß die oben beschriebenen Nachteile der gleichzeitigen Anwesenheit beider Komponenten wirksam vermieden werden.

Die Sterilisation der erfindungsgemäßen Einzellösungen ist ebenfalls unproblematisch, da festgestellt wurde, daß Glucose bei einem pH-Wert unter 4,0 problemlos sterilisiert werden kann. Der geringe pH-Wert führt dazu, daß weniger Glucoseabbauprodukte gebildet werden. Auf diese Weise können aufgrund der geringeren Konzentration von Abbauprodukten Einlaufschmerzen bei Dialysepatienten vermieden oder wesentlich verringert werden, d.h. die aus den erfindungsgemäßen Einzellösungen hergestellte Dialyselösung weist eine hohe Biokompatibilität auf. Der in der dritten Einzellösung vorliegende Puffer besitzt einen pH-Wert von etwa 7,2 bis 7,4. Die Pufferlösung enthält Bicarbonatlösung, wobei der Gehalt an Bicarbonat in der Mischlösung unter 40mM liegt. Vorzugsweise wird der Puffer aus HCO₃⁻ sowie aus einem Salz einer schwachen Säure hergestellt.

Besonders vorteilhaft ist es, wenn die dritte Einzellösung Bicarbonat aufweist, deren Gehalt bei maximal 10 mmol/l liegt. Hierdurch wird es möglich, den Druck des CO₂ innerhalb des entsprechenden Kompartiments so gering zu halten, daß keine besondere CO₂-Barriere gegen das Entweichen von CO₂ vorgesehen werden muß. Vielmehr ist der Einsatz einer normalen Polyolefinfolie bzw. einer normalen PVC-Folie ausreichend, um die Bicarbonatkonzentration konstant zu halten. Der Puffer der dritten Einzellösung weist zusätzlich ein Salz einer schwachen Säure, vorzugsweise Lactat, auf, dessen Pufferkapazität in dem kombinierten Puffer von Bicarbonat unterstützt wird.

In weiterer Ausgestaltung der vorliegenden Erfindung ist vorgesehen, daß die zweite Einzellösung keine Glucose aufweist. In diesem Fall ist die Glucose nur in der ersten Einzellösung vorgesehen und wird durch Mischung dieser Lösung mit der zweiten Einzellösung in der gewünschten Konzentration dosiert. Ebenso ist es selbstverständlich möglich, daß die zweite Einzellösung ebenfalls Glucose, jedoch in einer anderen Konzentration als die erste Einzellösung aufweist.

Besonders vorteilhaft ist es, wenn die erste und die zweite Einzellösung einen pH-Wert von 2,8 bis 4,0, vorzugsweise einen pH-Wert von 3,7 aufweisen und wenn die dritte Einzellösung einen pH-Wert von 8,5 bis 9,0 aufweist, so daß sich bei einer Mischung im Verhältnis fünf Teile der dritten Einzellösung zu 13 Teilen einer beliebigen Mischung der ersten und der zweiten Einzellösung in der fertigen Lösung ein pH-Wert von 6,8 bis 7,4 einstellt.

In weiterer Ausgestaltung der vorliegenden Erfindung ist vorgesehen, daß in der ersten sowie in der zweiten Einzellösung Calcium-, Natrium-, Magnesium- und H⁺-Überschußionen, Chloridionen sowie Glucose enthalten sind.

In der dritten Einzellösung sind Natriumionen sowie ein oder mehrere Salze schwacher Säuren enthalten. Derartige Salze umfassen Pyruvat-, α-Ketoglutarat-, Lactat- und Hydrogencarbonationen.

Besonders vorteilhaft ist es, wenn die physiologisch verträgliche Säure Salzsäure ist.

In einer bevorzugten Ausgestaltung der vorliegenden Erfindung umfassen die erste sowie die zweite Einzellösung folgende Bestandteile:
- Natrium:: 120 bis 140 mmol/l, vorzugsweise 133 mmol/l
- Calcium:: 1-3 mmol/l, vorzugsweise 1,7 oder 2,4 mmol/l
- Magnesium:: 0,5 bis 1,0 mmol/l, vorzugsweise 0,7 mmol/l
- H⁺-Überschuß:: 0,9 bis 1,1 mmol/l, vorzugsweise 1,0 mmol/l
- Chlorid:: 130 bis 150 mmol/l, vorzugsweise 140 mmol/l.

Die erste Einzellösung weist zusätzlich Glucose in einer Konzentration von 200 bis 1.000 mmol/l, vorzugsweise 500 mmol/l
und die zweite Einzellösung keine Glucose auf.

Die dritte Einzellösung weist vorteilhaft folgende Bestandteile auf:
- Natrium:: 120 bis 140 mmol/l, vorzugsweise 136 mmol/l
- Lactat:: 100 bis 140 mmol/l, vorzugsweise 126 mmol/l
- Hydrogencarbonat:: 5 bis 10 mmol/l, vorzugsweise 10 mmol/l.

Im übrigen hängen die verträglichen Konzentrationen vom Mischungsverhältnis der Summe der ersten und zweiten Lösung zur dritten Einzellösung ab. In der bevorzugten Ausgestaltung werden 5 Teile der ersten, 8 Teile der zweiten und 5 Teile der dritten Einzellösung gemischt.

In weiterer Ausgestaltung der vorliegenden Erfindung ist vorgesehen, daß die wenigstens drei Einzellösungen in einem Mehrkammerbeutel gelagert sind. Dabei kann der Mehrkammerbeutel als Kunststoffbeutel ausgeführt sein, der für jede der Einzellösungen eine Kammer aufweist.

Besonders vorteilhaft ist es, wenn die dritte Einzellösung das Salz einer schwachen Säure enthält. Die schwache Säure weist vorteilhaft einen pKa-Wert < 5 auf.

Die vorliegende Erfindung betrifft ferner ein Verfahren zur Herstellung einer Lösung nach einem oder mehreren der Ansprüche 1 bis 12, bei dem die Einstellung des gewünschten Mischungsverhältnisses automatisch durch die Dialysemaschine oder den Peritonealdialyse-Cycler erfolgt.

Weitere Vorteile und Einzelheiten der vorliegenden Erfindung werden anhand eines Ausführungsbeispiels näher erläutert.

Zur Herstellung der ersten und zweiten Einzellösung werden 7,784 g Natriumchlorid, 0,3557 g Calciumchlorid x 2H₂O, 0,1407 g Magnsiumchlorid x 6H₂O und 0,130 ml 25%ige Salzsäure in Wasser gelöst und auf 1000 ml aufgefüllt. In eine der beiden Lösungen werden zusätzlich 99 g Glucose-Monohydrat zugesetzt. Eine notwendige Korrektur des pH-Wertes auf < 4.0 kann durch Hinzufügen bzw. Weglassen von 25%iger Salzsäure oder Natriumhydroxid erfolgen. Beide Lösungen werden durch Membranvorfilter und anschließend durch Membransterilfilter in einen Kühltank filtriert. Nach Ansatzkontrolle und Freigabe der Lösungen werden diese in einen Mehrkammermehrschichtfolienbeutel gefüllt und mit Konnektoren verschlossen. Der trockene Beutel wird anschließend in einen Umbeutel umverpackt und dann hitzesterilisiert bei 121° C.

Zur Herstellung der dritten Einzellösung wird in 977 ml Wasser für Injektionszwekke, das auf 12 bis 14° C gekühlt wird 28,25 g Natriumlactat als 50%ige Lösung und 0,840 g Natriumhydrogencarbonat unter langsamen Rühren gelöst. Die Temperatur der Lösung soll während der Ansatz- und Lagerzeit 20° C nicht überschreiten. Die Lösung wird anschließend durch Membranvorfilter und Membransterilfilter in einen Kühltank filtriert. Nach Durchführung der Ansatzkontrolle und Freigabe der Lösung wird diese in den Mehrkammerbeutel gefüllt und mit Konnektoren verschlossen. Der trockene Beutel wird mit einem Umbeutel umverpackt. Anschließend wird bei 121 ° C sterilisiert.

Für den Einsatz werden die erste und die zweite Einzellösung in einem gewünschten Verhältnis miteinander gemischt. Die Mischung kann zeitlich konstant oder auch zur Erzielung eines Konzentrationsprofils zeitlich variabel erfolgen. Das Gemisch aus erster und zweiter Einzellösung wird im Verhältnis 13:5 mit der dritten Einzellösung gemischt.

Alle Einzellösungen liegen in getrennten Kompartimenten vor, die eine Verbindung zueinander bilden können, bzw. deren Ausläufe in eine gemeinsame Leitung oder Mischkammer münden. In dieser Verbindung/Ausleitung ist ein wiederverschließbares Strömungsregelelement vorgesehen, so daß die Menge der Einzellösungen eingestellt und während des Einsatzes auch individuell variiert werden kann. Ein Strömungsregelelement kann beispielsweise eine Rollenklemme oder eine okkludierende Pumpe sein.

## Patentansprüche

1. Lösung, insbesondere für die Hämo- oder Peritonealdialyse, bestehend aus wenigstens drei Einzellösungen, die nach einer Hitzesterilisation zusammengeführt und verabreicht werden,
wobei die erste Einzellösung Calciumionen, Elektrolytsalze sowie optional Glucose in einer Konzentration von 0-1000 mM enthält und mit einer physiologisch verträglichen Säure auf einen pH-Wert unter 4,0 angesäuert ist,
wobei die zweite Einzellösung optional Glucose enthält und sich die Glucose-konzentration in der ersten Einzellösung von der in der zweiten Einzellösung unterscheidet, und wobei die zweite Einzellösung alle weiteren Komponenten der ersten Einzellösung in gleicher Konzentration wie in dieser enthält
und wobei die dritte Einzellösung einen Puffer im physiologischen Bereich enthält, welcher Bicarbonat, Natriumionen und die Salze ein oder mehrerer schwacher Säuren, die Pyruvat-, α-Ketoglutarat-, Lactat- und Hydrogencarbonationen umfassen, aufweist.

2. Lösung nach Anspruch 1, **dadurch gekennzeichnet, daß** die zweite Einzellösung keine Glucose aufweist.

3. Lösung nach einem oder mehreren der Ansprüche 1 bis 2, **dadurch gekennzeichnet, daß** die erste und die zweite Einzellösung einen pH-Wert von 2,8 bis 4,0, vorzugsweise einen pH-Wert von 3,7 aufweisen und daß die dritte Einzel-lösung einen pH-Wert von 8,5 bis 9,0 aufweist, so daß sich bei einer Mischung im Verhältnis fünf Teile der dritten Einzellösung zu 13 Teilen einer beliebigen Mischung der ersten und zweiten Einzellösung in der fertigen Lösung ein pH-Wert von 6,8 bis 7,4 einstellt.

4. Lösung nach einem oder mehreren der Ansprüche 1 und 3, **dadurch gekennzeichnet, daß** in der ersten sowie in der zweiten Einzellösung Calcium-, Natrium-, Magnesium-, H⁺-Überschußionen, Chloridionen sowie Glucose enthalten sind.

5. Lösung nach einem oder mehreren der Ansprüche 1 bis 4, **dadurch gekennzeichnet, daß** in der dritten Einzellösung Natriumionen enthalten sind.

6. Lösung nach einem oder mehreren der Ansprüche 1 bis 5, **dadurch gekennzeichnet, daß** die physiologisch verträgliche Säure Salzsäure ist.

7. Lösung nach einem oder mehreren der Ansprüche 1 bis 3, 5 und 6, **dadurch gekennzeichnet,**
**daß** die erste sowie die zweite Einzellösung folgende Bestandteile umfassen:
Natrium: 120-140 mmol/l, vorzugsweise 133 mmol/l
Calcium: 1-3 mmol/l, vorzugsweise 1,7 oder 2,4 mmol/l
Magnesium: 0,5-1,0 mmol/l, vorzugsweise 0,7 mmol/l
H⁺-Überschuß: 0,9-1,1 mmol/l, vorzugsweise 1,0 mmol/l
Chlorid: 130-150 mmol/l, vorzugsweise 140 mmol/l,
**daß** die erste Einzellösung zusätzlich Glucose in einer Konzentration von 200-1000 mmol/l, vorzugsweise 500 mmol/l enthält
und **daß** die zweite Einzellösung keine Glucose aufweist.

8. Lösung nach einem oder mehreren der Ansprüche 1 bis 7, **dadurch gekennzeichnet, daß** die dritte Einzellösung folgende Bestandteile umfaßt:
Natrium: 120-140 mmol/l, vorzugsweise 136 mmol/l
Lactat: 100-140 mmol/l, vorzugsweise 126 mmol/l
Hydrogencarbonat: 5-10 mmol/l, vorzugsweise 10 mmol/l

9. Lösung nach einem oder mehreren der Ansprüche 1 bis 8, **dadurch gekennzeichnet, daß** die wenigstens drei Einzellösungen in einem Mehrkammerbeutel gelagert sind.

10. Lösung nach einem oder mehreren der Ansprüche 1 bis 9, **dadurch gekennzeichnet, daß** die dritte Einzellösung das Salz einer schwachen Säure enthält.

11. Verfahren zur Herstellung einer Lösung nach einem oder mehreren der Ansprüche 1 bis 10, **dadurch gekennzeichnet, daß** die Einstellung des gewünschten Mischungsverhältnisses automatisch durch die Dialysemaschine oder den Peritonealdialyse-Cycler erfolgt.

## Claims

1. Solution, in particular for haemodialysis or peritoneal dialysis, consisting of at least three individual solutions which are combined and administered after a heat sterilization,
where the first individual solution comprises calcium ions, electrolyte salts and optionally glucose in a concentration of 0-1000 mM and is acidified with a physiologically tolerated acid to a pH below 4.0,
where the second individual solution optionally comprises glucose and the glucose concentration in the first individual solution differs from that in the second individual solution, and where the second individual solution comprises all further components of the first individual solution in the same concentration as in the latter
and where the third individual solution comprises a buffer in the physiological range, which includes bicarbonate, sodium ions, and the salts of one or more weak acids comprising pyruvate ions, α-ketoglutarate ions, lactate ions and hydrogen carbonate ions.

2. Solution according to Claim 1, **characterized in that** the second individual solution includes no glucose.

3. Solution according to one or more of Claims 1 to 2, **characterized in that** the first and the second individual solution have a pH of from 2.8 to 4.0, preferably a pH of 3.7, and **in that** the third individual solution has a pH of from 8.5 to 9.0, so that a pH of from 6.8 to 7.4 is set up in the finished solution on mixing in the ratio of five parts of the third individual solution to 13 parts of any mixture of the first and second individual solution.

4. Solution according to one or more of Claims 1 and 3, **characterized in that** the first and the second individual solution comprise calcium ions, sodium ions, magnesium ions, H⁺ excess ions, chloride ions and glucose.

5. Solution according to one or more of Claims 1 to 4, **characterized in that** the third individual solution comprises sodium ions.

6. Solution according to one or more of Claims 1 to 5, **characterized in that** the physiologically tolerated acid is hydrochloric acid.

7. Solution according to one or more of Claims 1 to 3, 5 and 6, **characterized**
**in that** the first and the second individual solution include the following ingredients:
sodium: 120-140 mmol/l, preferably 133 mmol/l
calcium: 1-3 mmol/l, preferably 1.7 or 2.4 mmol/l
magnesium: 0.5-1.0 mmol/l, preferably 0.7 mmol/l
H⁺ excess: 0.9-1.1 mmol/l, preferably 1.0 mmol/l
chloride: 130-150 mmol/l, preferably 140 mmol/l,
**in that** the first individual solution additionally comprises glucose in a concentration of 200-1000 mmol/l, preferably 500 mmol/l,
and **in that** the second individual solution includes no glucose.

8. Solution according to one or more of Claims 1 to 7, **characterized in that** the third individual solution includes the following ingredients:
sodium: 120-140 mmol/l, preferably 136 mmol/l
lactate: 100-140 mmol/l, preferably 126 mmol/l
hydrogen carbonate: 5-10 mmol/l, preferably 10 mmol/l.

9. Solution according to one or more of Claims 1 to 8, **characterized in that** the at least three individual solutions are stored in a multichamber bag.

10. Solution according to one or more of Claims 1 to 9, **characterized in that** the third individual solution comprises the salt of a weak acid.

11. Method for preparing a solution according to one or more of Claims 1 to 10, **characterized in that** the desired mixing ratio is adjusted automatically by the dialysis machine or the peritoneal dialysis cycler.

## Revendications

1. Solution, en particulier une solution d'hémodialyse ou de dialyse péritonéale, consistant en au moins trois solutions individuelles, qui sont rassemblées après une stérilisation thermique et administrées,
où la première solution individuelle contient des ions calcium, des sels d'électrolyte ainsi que le cas échéant, du glucose en des concentrations de 0-1000 mM et est rendue acide avec un acide physiologiquement compatible, à un pH inférieur à 4,0,
où la deuxième solution individuelle contient le cas échéant, du glucose, où la concentration du glucose dans la deuxième solution individuelle est différente de celle dans la première solution individuelle, et où la deuxième solution individuelle contient tous les autres composants de la première solution individuelle en la même concentration que dans celle-ci, et
où la troisième solution individuelle contient un tampon dans l'intervalle physiologique, le tampon comprenant du bicarbonate, des ions sodium et les sels d'un ou de plusieurs acides faibles qui comprennent des ions pyruvate, α-cétoglutarate, lactate et hydrogénocarbonate.

2. Solution selon la revendication 1, **caractérisée en ce que** la deuxième solution individuelle ne contient pas de glucose.

3. Solution selon l'une ou plusieurs des revendications 1 à 2, **caractérisée en ce que** la première et la deuxième solutions individuelles présentent un pH allant de 2,8 à 4,0, de préférence un pH de 3,7 et **en ce que** la troisième solution individuelle présente un pH de 8,5 à 9,0, de sorte que lors du mélange en un rapport de 5 parties de la troisième solution individuelle à 13 parties d'un mélange quelconque des première et la deuxième solutions individuelles, on ajuste dans la solution finale, un pH allant de 6,8 à 7,4.

4. Solution selon l'une ou plusieurs des revendications 1 à 3, **caractérisée en ce que** dans la première et la deuxième solutions individuelles, sont présents des ions calcium, sodium, magnésium, H⁺ en excès, des ions chlorure ainsi que du glucose.

5. Solution selon l'une ou plusieurs des revendications 1 à 4, **caractérisée en ce que** dans la troisième solution individuelle, sont présents des ions sodium.

6. Solution selon l'une ou plusieurs des revendications 1 à 5, **caractérisée en ce que** l'acide physiologiquement compatible est l'acide chlorhydrique.

7. Solution selon l'une ou plusieurs des revendications 1 à 3, 5 et 6, **caractérisée en ce que** la première ainsi que la deuxième solutions individuelles comprennent les constituants suivants:
Sodium: 120 à 140 mmoles/litre, de préférence 133 mmoles/litres
Calcium: 1-3 mmoles/litre, de préférence 1,7 à 2,4 mmoles/litres
Magnésium: 0,5 à 1,0 mmole/litre, de préférence 0,7 mmole/litres
Excès de H+: 0,9 à 1,1 mmole/litre, de préférence 1,0 mmole/litres
Chlorure: 130 à 150 mmoles/litre, de préférence 140 mmoles/litres,
**en ce que** la première solution individuelle présente en outre, du glucose en une concentration de 200 à 1000 mmoles/litre, de préférence de 500 mmoles/litre,
et **en ce que** la deuxième solution ne contient pas de glucose.

8. Solution selon l'une ou plusieurs des revendications 1 à 7, **caractérisée en ce que** la troisième solution individuelle comprend les constituants suivants:
Sodium: 120 à 140 mmoles/litre, de préférence 136 mmoles/litres
Lactate: 100 à 140 mmoles/litre, de préférence 126 mmoles/litres
Hydrogénocarbonate: 5 à 10 mmoles/litre, de préférence 10 mmoles/litres.

9. Solution selon l'une ou plusieurs des revendications 1 à 8, **caractérisée en ce que** les au moins trois solutions individuelles sont stockées dans une poche à plusieurs chambres.

10. Solution selon l'une ou plusieurs des revendications 1 à 9, **caractérisée en ce que** la troisième solution individuelle contient le sel d'un acide faible.

11. Procédé de préparation d'une solution selon l'une ou plusieurs des revendications 1 à 10, **caractérisé en ce que** l'ajustement des rapports de mélange souhaités est réalisé automatiquement par la machine de dialyse ou le cycleur de dialyse péritonéale.
